Europäisches Patentamt

European Patent Office

Office européen des brevets

(19)

(11) Publication number: **0 040 238**
**B1**

(12) **EUROPEAN PATENT SPECIFICATION**

(45) Date of publication of patent specification: **11.07.84**

(21) Application number: **80902378.1**

(22) Date of filing: **29.10.80**

(86) International application number:
**PCT/US80/01450**

(87) International publication number:
**WO 81/01417 28.05.81 Gazette 81/13**

(51) Int. Cl.³: **C 12 N 9/48, C 12 N 9/72,
A 61 K 43/00**

(54) PLASMINOGEN ACTIVATORS USEFUL AS THERAPEUTIC AND DIAGNOSTIC AGENTS AND THEIR ISOLATION.

(30) Priority: **13.11.79 US 93246**

(43) Date of publication of application:
**25.11.81 Bulletin 81/47**

(45) Publication of the grant of the patent:
**11.07.84 Bulletin 84/28**

(84) Designated Contracting States:
**AT CH DE FR GB LI NL SE**

(56) References cited:
**FR - A - 2 387 242**
**GB - A - 1 068 817**
**LU - A - 80 716**
**US - A - 2 961 382**
**US - A - 2 983 647**
**US - A - 2 989 440**
**US - A - 3 042 667**
**US - A - 3 140 984**
**US - A - 3 256 158**
**US - A - 3 723 251**
**US - A - 3 755 083**
**US - A - 3 812 245**

(73) Proprietor: HUSAIN, Syed Shaukat
139 Upland Avenue
Newton Highland, MA (US)
(73) Proprietor: LIPINSKI, Boguslaw
97 Beaumount Avenue
Newtonville, MA (US)
(73) Proprietor: GUREWICH, Victor
300 Mt, Auburn Street Suite 309
Cambridge, MA 2109 (US)

(72) Inventor: HUSAIN, Syed Shaukat
139 Upland Avenue
Newton Highland, MA (US)
Inventor: LIPINSKI, Boguslaw
97 Beaumount Avenue
Newtonville, MA (US)
Inventor: GUREWICH, Victor
300 Mt, Auburn Street Suite 309
Cambridge, MA 2109 (US)

(74) Representative: Heidrich, Udo, Dr. jur., Dipl.-Phys.
Franziskanerstrasse 30
D-8000 München 80 (DE)

(56) References cited:
**US - A - 3 933 996**
**US - A - 3 939 258**

Courier Press, Leamington Spa, England.

US - A - 3 957 582
US - A - 4 025 390
US - A - 4 036 945
US - A - 4 094 965
US - A - 4 160 697

Lubert Stryer BIOCHEMISTRY (1975/1981) Page
105
CHEMICAL ABSTRACTS, vol. 89, no. 7, 14
August 1978, abstract no. 57189k, pages 91-
102 Columbus, Ohio (US) P. WALLEN:
"Activation of plasminogen with urokinase and
tissue activator"

## Description

Background of the invention

This invention relates to the preparation of plasminogen activators useful as therapeutic and diagnostic agents.

It has been recognized that plasminogen activators are useful in the treatment of blood clots. By introduction of such an activator to a human blood stream in sufficient quantities and duration, a blood clot that has occurred can be dissolved.

The procedures used in the past for isolating plasminogen activators have been characterized by their complexity and costliness. In the case, for instance, of urokinase, the plasminogen activator found in urine, the yield of the known commercial process is so low that a great quantity of urine must be collected and processed in order to treat a single patient. Isolation of urokinase from other sources, as from kidney tissue culture medium, or isolation of other plasminogen activators, such as streptokinase, likewise involves many steps, which are either costly, or may be associated with adverse immunological reactions. Moreover, the commercially available activators for clinical use (Abbokinase and Streptokinase) are known to have a low affinity for fibrin clots and therefore may not be optimally effective or may be associated with adverse side effects due to generalized proteolysis.

References regarding prior isolation of urokinase are the following:

(a) White, W. F.; Barlow, G. H.; Mozen, M. M.; The Isolation and Characterization of Plasminogen Activators [Urokinase] from Human Urine. Biochemistry, Vol. 5, pp. 2,160—2,169, 1966;

(b) Pye, E. K.; Maciag, T.; Kelly, P.; Iyznger, M. R.; Purification of Urokinase by Affinity Chromotography In: *Thrombosis and Urokinase*, Eds. R. Paoletti and S. Sherry. Academic Press, London, New York, San Francisco 1977.

FR—A—23 87 242 describes the separation of 33,000 Daltons mw urokinase from 54,000 Daltons mw urokinase, using an ion exchange resin; table IV (p. 17) gives activities in the fibrin plate test of the two urokinases of 170,000 and 128,000 CTA units, respectively.

Summary of the invention

The present invention provides a new class of therapeutic and diagnostic agents in the form of a plasminogen activator characterized by strong affinity for fibrin. Also, the invention overcomes the above problems and makes it possible to isolate relatively large quantities of plasminogen activator from comparatively small quantities of starting material.

The inventors have provided, as a novel thrombolytic agent, high mw single chain urokinase isolated from a biological source. Previously available urokinase thrombolytic agents are of a two chain structure (or a single chain degradation product of such two chain structure).

The high mw single chain form, although exhibiting, in the fibrin plate test, lower activity, has been found to have a high relative specificity for (i.e., preference for reaction with) the intended target, fibrin clots. In contrast, two chain urokinase, lacking such specificity, indiscriminately breaks down many desired components of the blood.

Inventors made their discovery by testing urokinase isolated by a fast, two-step procedure (see method claims).

Apparently, the prior art commerical isolation procedures have caused cleavage of a peptide bond, resulting in a two chain structure. This has apparently exposed the active site for undesirable reaction with blood components. In the different conformation of the high mw single chain molecule, the active site is in a relatively unavailable position for such reaction. Nevertheless, upon reaching the fibrin clot, the high mw single chain molecule becomes activated, presumably due then to a conformational change, triggered by the conditions at the fibrin clot.

Such an activating mechanism has been previously known with enzymes. Thus, Lubert Stryer, Biochemistry, 1975, 1981, describes enzymes that occur in an inactive precursor form, and which have been observed to be activated at a physiologically appropriate place. Indeed, such properties have been known in the case of plasminogen/plasmin, an enzyme system which also has a role in the lysing of fibrin clots.

According to the present invention, the new product is high affinity plasminogen activator isolated from a biological source such as urine and culture medium. An improved thrombolytic agent according to the invention consists essentially of urokinase that has high affinity for fibrin, and made available to the physician in lyophilized form.

Specifically a new product is provided comprising a plasminogen activator isolated from a biological source such as urine or tissue culture. The particular characteristics of this new product include a molecular weight of about 56,000 Daltons, a specific activity of 40,000—50,000 CTA units/mg when assayed on a fibrin plate, and the appearance of a single chain structure corresponding to a molecular weight of 56,000 Daltons as evidenced by a single protein band on 7.5 percent polyacrylamide gel when sodium dodecyl sulfate-gel electrophoresis is performed on the new product in the unreduced state. The product exhibits the appearance of a single chain structure as evidenced by a single band on 7.5 percent polyacrylamide gel when sodium dodecyl sulfate-gel electrophoresis is performed on the product which has been reduced by 0.1 M dithiothreitol. Most importantly, the new product displays a substantial binding affinity for fibrin as evidenced by its high affinity for fibrin.

A plasminogen activator tracer for detecting blood clots according to the invention consists essentially of a high affinity plasminogen activator to which is attached a radioactive label, such as Iodine or Technitium. The characteristics of the high affinity plasminogen activator include a molecular weight of about 56,000 Daltons, a specific activity of 40,000—50,000 CTA units/mg when assayed on a fibrin plate, and the appearance of a single chain structure corresponding to a molecular weight of 56,000 Daltons as evidenced by a single protein band on 7.5 percent polyacrylamide gel when sodium dodecyl sulfate-gel electrophoresis is performed on the new product in the unreduced state. The plasminogen activator retains the appearance of a single chain structure as evidenced by a single band on 7.5 percent polyacrylamide gel when sodium dodecyl sulfate-gel electrophoresis is performed on the plasminogen activator which has been reduced by 0.1 M dithiothreitol. Most importantly, the plasminogen activator displays a substantial binding affinity for fibrin as evidenced by its high affinity for fibrin celite. The high affinity that the plasminogen activator has for fibrin is the property that makes it, when radiolabelled, a new diagnostic agent for specific detection of fibrin thrombi through nuclear scanning.

According to the present invention the plasminogen activator product is isolated by providing a solid matrix, (preferably diatomaceous earth), having fibrin bonded thereto, exposing a liquid from a biological source to the fibrin-containing matrix, whereby plasminogen activator molecules which have high affinity for fibrin are bound to molecules of fibrin, removing the remaining liquid, and separating the plasminogen activator from the fibrin.

The invention is based on the discovery that fibrin precipitated on a solid adsorptive matrix retains considerable affinity for certain plasminogen activators especially for a species of urokinase here designated as, having "high affinity". In contrast, if fibrin is covalently attached to activated agarose (i.e. agarose activated by cyanogen bromide) such affinity for the plasminogen activator does not then occur. While the cause for this difference is not experimentally proven, we now believe that fibrin precipitated on an adsorptive matrix without covalent bonding leaves free certain $\varepsilon$-amino groups of lysine residues of the fibrin. These $\varepsilon$-amino groups of lysine residues are believed to contribute to the newly-discovered affinity. In contrast, if the fibrin is covalently attached to activated agarose, the $\varepsilon$-amino groups of the lysine residues may be covalently linked to the agarose in such a manner as to substantially block the affinity. It is apparently for such reasons that the existence of a high affinity urokinase has been overlooked.

In preferred embodiments of the method of isolation of the invention a number of further conditions are preferred. The plasminogen activator is exposed to the fibrin-containing matrix by stirring fibrin-solid particles into a batch of liquid in which the plasminogen activator is present. The activator bound to the fibrin that is precipitated on the solid matrix is eluted from the fibrin surface. The eluate containing plasminogen activator eluted from the fibrin surface is passed through a gel filtration column to separate the high affinity plasminogen activator from the eluting agents and fibrin contaminants.

Preferably, for providing the solid matrix with fibrin on its surface, fibrinogen is treated with thrombin in the presence of the matrix in a manner to precipitate fibrin upon the matrix. Preferably this is done by exposing the adsorptive matrix surface to excess fibrinogen in a buffer, in quantity sufficient to effectively cover all adsorptive surface, and thereafter introducing thrombin in a buffer to convert fibrinogen to fibrin, whereby the adsorptive surface can be effectively fully occupied by fibrin.

Furthermore, according to a specific aspect of the invention, for isolating high affinity urokinase, the invention comprises providing an adsorptive solid matrix, preferably diatomaceous earth particles, precipitating fibrin on its surface by treating fibrinogen with thrombin in the presence of the matrix, exposing urine or a culture medium to the fibrin-containing matrix whereby urokinase of the species which has the discovered high affinity towards fibrin is bound to it, removing the unbound material with a buffer solution, separating the high affinity urokinase from the fibrin first by eluting the urokinase from the fibrin surface by an eluant agent containing a member from the group consisting of arginine, lysine and $\varepsilon$-amino-caproic acid in an aqueous solution and thereafter separating the urokinase from the agent.

In this method preferably the urine or culture medium is exposed to the firbin-carrying matrix by mixing particles with the urine or tissue culture medium and the liquid is removed by decantation and filtration followed by repeated washing of the fibrin matrix and bound activator with a buffer.

General principles

The procedure utilizes properties of certain plasminogen activators to bind to fibrin. This principle is applied, in the example below, to the isolation of a plasminogen activator from urine which has a high affinity towards fibrin (High Affinity Urokinase, HAUK), but is also applicable to the extraction of high affinity plasminogen activators from other sources such as kidney tissue culture medium extracts.

The procedure utilizes a matrix consisting of material with the property to adsorb fibrinogen. In the preferred example below, diatomaceous earth, Celite®, was shown to be suitable for this purpose. A powdered form of this material is used in order to provide a large surface area. After this matrix is mixed with fibrinogen, the latter is converted to fibrin by exposure to thrombin, resulting in the precipitation of fibrin on the matrix. This particular matrix circumvents the formation of a gel which normally forms when fibrinogen is exposed to thrombin. When fibrin-Celite is stirred in urine, the HAUK is bound to the affinity matrix. Subsequently, the bound activator is removed from fibrin-Celite by washing with an eluant. The eluted HAUK is contaminated only by a small amount of fibrin, washed off from the column by the eluant, which along with the eluant material is easily separated by gel filtration. The final solution containing the activator is then lyophilized.

## Method

10 g powdered diatomaceous earth (Celite Analytical filter aid, Fisher Scientific Co.), the matrix, is washed with distilled water and mixed with 2 percent human fibrinogen (Kabi, Stockholm, Sweden) in 25 ml buffer (0.05 M sodium phosphate, pH 7.4 containing 0.1 M NaCl and 1 m M EDTA). Thrombin (Parke Davis, Bovine topical) 100 u in 1 ml of buffer is added with constant stirring at room temperature to ensure good mixing to prevent agglutination of the fibrin-matrix. After 30 minutes, the fibrin-matrix is filtered through a sintered glass funnel and washed thoroughly with the same buffer (1 liter) followed by a buffer containing 0.2 M arginine. A final washing is performed with 0.05 M sodium phosphate buffer pH 7.4 containing 1 m M EDTA and 0.3 M NaCl. The washed fibrin-matrix (15 ml of the settled volume) is added to 1 liter of fresh human urine and the mixture stirred for one hour in cold (4°C). After filtration, the fibrin-matrix is packed in a column, washed with the equilibration buffer (8—10 column volumes). The activator is then eluted from the fibrin with arginine (0.2 M in the above buffer) in a single sharp peak. Alternatively, elution may be accomplished with lysine or ε-amino-caproic acid. The arginine and any fibrin derivatives contaminating the activator are then removed by gel filtration on Sephadex G-100 (Pharmacia Chemicals, Upsala, Sweden) and then the solution containing the activator is lyophilized.

## Results

Approximately 12.00—30.00 CTA units of HAUK are obtained from 1 liter of fresh human urine. The molecular weight of HAUK was determined to be approximately 56,000 Daltons by gel-filtration on Sephadex G-200 superfine [For technique, see P. Andrews, Methods Biochem. Anal. 8, 1—53 (1970)]. The HAUK material converted plasminogen to plasmin as demonstrated by a chromogenic synthetic substrate. On a plasminogen-rich fibrin plate, it induced rapid lysis of fibrin. It displays a specific activity of 40,000—50,000 CTA units/mg when assayed on the fibrin plate. [For technique, see P. Brakman, "Fibrinolysis: A Standardized Fibrin Plate Method and a Fibrinolytic Assay of Plasminogen" published by Scheltima and Holkema, Amsterdam, pp. 1—124 (1967)].

Comparison of HAUK with commercial urokinase (Abbott)

Two principle differences and one common property were found which are summarized in the table below:

## TABLE

|  | Urokinase (Abbott) | High affinity urokinase HAUK (present invention) |
|---|---|---|
| Molecular weight (Daltons) | 36,000 | 56,000 |
| Fibrin binding (fibrin-celite column) | <5% | 100% |
| Binding to sepharose-fibrin | <1% | <1% |

In addition, more effective lysis of fibrin under physiological conditions was found for HAUK compared to the commercial urokinase.

The commercial urokinase (Abbott) contains predominantly of the low molecular weight form. The fraction of high molecular weight urokinase found in the commercial urokinase (Abbott) appears to be a two chain structure as evidenced by two protein bands after reduction and sodium dodecyl sulfate-gel electrophoresis [for technique see K. Weber and M. Osborn, *The Proteins*, eds. Neurath, H. and Hill, L. L. (Academic Press, New York) Vol. 1. pp. 179— 223 (1975)]. This high molecular weight form of urokinase has low affinity for fibrin whereas the low molecular weight urokinase has none. Cumulatively, the commercial urokinase mixture of high and low molecular weight urokinases are known to have a very low affinity for fibrin, as noted above.

HAUK has a molecular weight of about 56,000 Daltons, as comparable to that of the high molecular weight form in commercial urokinase. However, HAUK appears as a one chain structure as evidenced by one protein band after reduction with 0.1 M dithiothreitol and sodium dodecyl sulfate-gel electrophoresis on 7.5 percent polyacrylamide gel. [*Ibid.*, Weber *et al.*]

Binding of HAUK, the high molecular weight single chain species of urokinase of the invention, to fibrin celite under conditions used for its adsorption indicates its high binding affinity for fibrin.

It is believed that HAUK, which we for the first time have isolated, retains the native molecular form. The few step, fast method of isolation enables the single chain molecular structure to be retained.

On the other hand, the relatively slow, many step procedures of the prior art, we believe, have resulted in degradation of the molecule, and have resulted in the two chain structure and low affinity for fibrin.

Features of the method

The present method has a number of advantages over existing procedures:

1. It is a rapid, single-step, instead of a lengthy, multi-step isolation procedure. The length procedure currently in use allows undesirable denturation, autodegradation, or degradation of urokinase by other proteolytic enzymes.
2. The method separates the high affinity from low affinity plasminogen activator, and establishes for the first time that fresh urine contains a high affinity urokinase (40 to 80 percent of the total urokinase, depending on the subject).
3. The method provides a high yield (about 60 percent) of the total HAUK in urine and with no low-affinity urokinase present. In contrast urokinase that is presently commercially available for clinical use (Abbokinase, Abbott) has essentially no HAUK and is a low-affinity plasminogen activator.

Applications

I. General

Because the described extraction method is economical and rapid, it should provide a relatively cheap and abundant supply of human plasminogen activator characterized by a high affinity for fibrin for both scientific research and clinical purposes.

II. Therapy

The two commercially available plasminogen activators Streptokinase (Hoechst and Kabi) and Urokinase (Abbott) which have received FDA approval for the thrombolytic therapy of pulmonary embolism are low affinity activators. As a result, a large amount of activator (100,000—200,000 CTA $\mu$/hour) must be infused to achieve clot lysis. Since plasmin is an enzyme with broad specificity, the effect of infusing these activators is an undesired state of generalized proteolysis which degrades several plasma protines (fibrinogen, factors V and VIII).

By contrast, the HAUK discovered and efficiently isolated according to the present invention, because of its affinity for fibrin, will provide a much more specific fibrinolysis at lower concentrations of the activator

III. Diagnosis

The fibrin binding properties of HAUK can also be utilized for the detection of intravascular clots or thrombi. By attaching an appropriate radioactive isotope (e.g. $^{125}$Iodine or Technitium) to the molecule and then by intravenous injection of the labelled HAUK, it is possible to identify intravascular fibrin by radionuclear scanning. For example, the labelled HAUK can be used for the specific detection of pulmonary emboli (lung clots) by lung scanning. This diagnosis is notoriously difficult to make by current methods because they are too non-specific.

Prior efforts to employ radioactively-labelled plasminogen activators for radionuclear scanning are believed to have been relatively unsuccessful because the plasminogen activators used lacked the essential property of having high affinity for fibrin. References regarding such prior efforts are (a) Millar, W. T. and Smith, J. F. B., Localization of Deepvenous Thrombosis Using Technitium 99 m—labelled Urokinase (preliminary communication), Lancet Vol. 2, p. 695—696, 1974; (b) Kempi, V.; Van DerLinden, W.; and van Scheele, C.; Diagnosis of Deep Vein Thrombosis with 99 MTc—streptokinase: A Clinical Comparison with Phlebography, British Medical Journal, Vol. 2, p. 748—749, 1974.

Claims

1. A thrombolytic agent

— comprising plasminogen activator
— isolated from a biological source such as urine or tissue culture and
— consisting essentially of urokinase, which is characterized as

a) having a molecular weight of about 56,000 Daltons,
b) having a specific activity of about 40,000—50,000 CTA units/mg
— when assayed on a fibrin plate,
c) exhibiting the appearance of a single chain structure
— corresponding to a molecular weight of 56,000 Daltons
— as evidenced by a single protein band on 7.5 percent polyacrylamide gel
— when sodium dodecyl sulfate-gel electrophoresis is performed on unreduced urokinase,
d) exhibiting the said appearance of a single chain structure

— as evidenced by a single protein band on 7.5 percent polyacrylamide gel
— when sodium dodecyl sulfate-gel electrophoresis is performed on urokinase
— which has been reduced by 0.1 m dithiothreitol.

2. The method of isolating the plasminogen activator of claim 1 characterized by the steps of providing a solid matrix having a fibrin bonded thereto,

exposing a liquid from a biological source containing high fibrin-affinity plasminogen activator to the fibrin-containing matrix, whereby those plasminogen activator molecules which have high affinity for fibrin are bound to molecules of fibrin,
removing the remaining liquid, and
separating the plasminogen activator from the fibrin.

3. The method of claim 2 wherein the plasminogen activator is eluted from the fibrin surface by an eluant agent solution containing a member of the group consisting of arginine, lysine, and ε-amino-caproic acid.

4. The method of claim 2 of 3 wherein said matrix comprises diatomaceous earth.

5. The method of any of the foregoing claims 2 to 4 wherein, for providing said matrix with fibrin said matrix has an adsorptive surface, fibrinogen is adsorbed upon the surface of said matrix and is thereafter treated with thrombin in the presence of the said matrix in a manner to cause fibrin to be precipitated upon said matrix without gel formation.

6. The method of claim 5 comprising exposing the adsorptive surface of said matrix to fibrinogen in quantity sufficient to effectively cover said adsorptive surface, thereafter introducing thrombin to covert said adsorbed fibrinogen to fibrin, whereby said adsorptive surface is effectively fully occupied by adsorbed fibrin.

7. The method of isolating the plasminogen activator of claim 1 characterized by the steps of

providing a solid adsorptive matrix with fibrin by treating fibrinogen with thrombin in the presence of said matrix to cause fibrin to be in an adsorbed state upon said matrix substrate,
exposing to the substrate a mother liquid containing urokinase to cause those molecules which have high affinity toward fibrin to be bound to the fibrin,
removing unbound material by washing with a buffer solution,
separating the urokinase from the fibrin first by eluting the urokinase from the fibrin surface by an eluant agent comprising a member of the group consisting of arginine, lysine, and ε-amino-caproic acid, and thereafter separating said urokinase from said agent.

8. The method of any of the foregoing claims 2—7 wherein said matrix comprises particles and wherein the liquid is exposed to said fibrin-carrying matrix by mixing said particles with said liquid.

9. The method of any of the foregoing claims 2—8 wherein said liquid is removed by decanting and filtering followed by repeated washing with a buffer.

10. A plasminogen activator enzyme concentrate produced by the method of any of the foregoing claims 2—9.

11. A therapeutic agent comprising the plasminogen activator enzyme concentrate of claim 1 or 10.

12. The plasminogen activator of any of the claims 1, 10 or 11

having a label whereby said enzyme is useful as a tracer, for example for detecting blood clots.

13. The plasminogen activator of claim 12 further characterized in that said label is provided by radioactive iodine or technetium bonded to said enzyme.

**Patentansprüche**

1. Thrombolytikum

— mit Plasminogen-Aktivator
— isoliert aus einer biologischen Quelle wie Urin oder Gewebekultur und
— im wesentlichen aus Urokinase bestehend, die gekennzeichnet ist durch
a) ein Molekulargewicht von ca. 56.000 Dalton,
b) eine spezifische Aktivität von ca. 40.000 bis 50.000 CTA-Einheiten/mg
— bei Prüfung auf einer Fibrin-Platte,
c) das Erscheinen einer einkettigen Struktur
— entsprechend einem Molekulargewicht von 56.000 Dalton
— wie nachgewiesen durch eine einzelne Protein-Bande auf 7,5% Polyacrylamid-Gel
— bei Natriumdodecylsulfat-Gel-Elektrophorese an unreduzierter Urokinase,
d) das Erscheinen einer einkettigen Struktur
— wie nachgewiesen durch eine einzelne Protein-Bande auf 7,5% Polyacrylamid-Gel
— bei Natriumdodecylsulfat-Gel-Elektrophorese an Urokinase,
— die durch 0,1 m Dithiothreitol reduziert worden ist.

2. Verfahren zum Isolieren des Plasminogen-Aktivators nach Anspruch 1, gekennzeichnet durch die folgenden Schritte:

— Vorsehen einer Feststoff-Matrix mit daran gebundenem Fibrin,
— Aufbringen einer Flüssigkeit von einer biologischen Quelle, die einen Plasminogen-Aktivator mit hoher Fibrin-Affinität enthält, auf die Fibrin-haltige Matrix, wodurch diejenigen Plasminogen-Aktivator-Moleküle, die hohe Auffinität für Fibrin besitzen, an die Fibrin-Moleküle gebunden werden,
— Entfernen der restlichen Flüssigkeit und
— Abtrennen des Plasminogen-Aktivators vom Fibrin.

3. Verfahren nach Anspruch 2, dadurch gekennzeichnet,

— daß der Plasminogen-Aktivator aus der Fibrin-Oberfläche durch eine Elutionsmittel-Lösung eluiert wird, die Arginin, Lysin oder ε-Amino-Capronsäure enthält.

4. Verfahren nach Anspruch 2 oder 3, dadurch gekennzeichnet,

— daß die Matrix Diatomeenerde enthält.

5. Verfahren nach einem der Ansprüche 2—4, dadurch gekennzeichnet,

— daß zum Vorsehen der Matrix mit Fibrin die Matrix eine Adsorptionsfläche besitzt, Fibrinogen an der Oberfläche der Matrix adsorbiert und anschließend mit Thrombin in Gegenwart der Matrix derart behandelt wird, daß Fibrin auf die Matrix ohne Gel-Bildung ausfällt.

6. Verfahren nach Anspruch 5, gekennzeichnet durch

— Beaufschlagen der Adsorptionsfläche der Matrix mit Fibrinogen in ausreichender Menge, um wirksam die Adsorptionsfläche zu bedecken, anschließendes Einführen von Thrombin, um das adsorbierte Fibrinogen in Fibrin umzuwandeln, wodurch die adsorptionsfläche im wesentlichen vollständig vom adsorbierten Fibrin besetzt wird.

7. Verfahren zum Isolieren des Plasminogen-Aktivators nach Anspruch 1, gekennzeichnet durch

— Vorsehen einer festen Adsorptions-Matrix mit Fibrin durch Behandeln von Fibrinogen mit Thrombin in Gegenwart der Matrix, damit Fibrin in adsorbiertem Zustand auf das Matrix-Substrat gelangt,
— Beaufschlagen des Substrats mit einer Mutterflüssigkeit, die Urokinase enthält, um diejenigen Moleküle, die hohe Affinität für Fibrin besitzen, am Fibrin zu binden,
— Entfernen nicht gebundenen Materials durch Waschen mit einer Pufferlösung, und
— Abtrennen der Urokinase vom Fibrin zuerst durch Eluieren der Urokinase von der Fibrin-Oberfläche durch ein Elutionsmittel, das Argenin, Lysin oder ε-Amino-Capronsäure besitzt, und anschließendes Abtrennen der Urokinase vom Mittel.

8. Verfahren nach einem der Ansprüche 2—7, dadurch gekennzeichnet,

— daß die Matrix Teilchen besitzt und
— daß die Flüssigkeit auf die Fibrin-tragende Matrix durch Mischen der Teilchen mit der Flüssigkeit aufgebracht wird.

9. Verfahren nach einem der Ansprüche 2—8, dadurch gekennzeichnet,

— daß die Flüssigkeit entfernt wird durch Dekentieren und Filtern sowie anschließendes wiederholtes Waschen mit einem Puffer.

10. Plasminogen-Aktivator-Enzym-Konzentrat, hergestellt durch das Verfahren nach einem der Ansprüche 2—9.

11. Therapeutisches Mittel

— mit dem Plasminogen-Aktivator-Enzym-Konzentrat nach Anspruch 1 oder 10.

12. Plasminogen-Aktivator nach einem der Ansprüche 1, 10 und 11,

— mit einem Label (Markierungsstoff), wodurch das Enzym als Tracer, z.B. zum Erfassen von Blut-Thromben, einsetzbar ist.

13. Plasminogen-Aktivator nach Anspruch 12, dadurch gekennzeichnet,

— daß der Label gebildet ist durch radioaktives Jod oder Technetium, gebunden an das Enzym.

## Revendications

1. Agent thrombolytique comprenant:

— un activateur plasminogène isolé à partir d'une source biologique, telle que l'urine ou une culture de tissue, et constitué essentiellement d'urokinase qui est caractérisé en ce que:
a) il a un poids moléculaire d'environ 56 000 daltons;
b) il a une activité spécifique d'environ 40 000—50 000 unités CTA/mg;

lorsqu'il est testé sur une plaque de fibrine;

c) il présente l'apparence d'une structure à chaîne unique

— correspondant à un poids moléculaire de 56 000 daltons,
— comme le montre la bande de protéine unique sur gel de polyacrylamide à 7,5%,
— lorsque l'électrophorèse est effectuée avec du gel de sulfate de dodécyl de sodium sur l'urokinase non réduite;

d) il présente l'apparence d'une structure à chaîne unique,
— comme le montre la bande de protéine unique sur gel de polyacrylamide à 7,5%,
— lorsqu'on effectue une électrophorèse avec du gel de sulfate de dodécyl de sodium sur de l'urokinase
— qui a été réduite par du dithiotreitol 0,1 m.

2. Procédé d'isolement d'activateur plasminogène selon la revendication 1, caractérisé par les étapes de fourniture d'une matrice solide ayant de la fibrine qui y est liée.

— d'exposition d'un liquide à partir d'une source biologique contenant un activateur plasminogène de haute affinité vis-à-vis de la fibrine à la matrice contenant la fibrine, ce par quoi les molécules de l'activateur plasminogène qui présente une haute affinité pour la fibrine sont liées aux molécules de fibrine,
— d'élimination du liquide restant
— et la séparation de l'activateur plasminogène à partir de la fibrine.

3. Procédé selon la revendication 2, caractérisé en ce que l'activateur plasminogène est élué à partir de la surface de la fibrine par une solution d'agents éluants contenant un membre du groupe constitué par l'arginine, la lysine et l'acide epsilon-amino caproïque.

4. Procédé selon la revendication 2 ou 3, caractérisé en ce que la matrice comprend de la terre de diatomées.

5. Procédé selon l'une quelconque des revendications 2 à 4, caractérisé en ce que pour fournir à ladite matrice de la fibrine, ladite matrice ayant une surface adsorbante, le fibrinogène est adsorbé sur la surface de ladite matrice et est ensuite traité avec de la thrombine en présence de ladite matrice de façon à faire précipiter la fibrine sur ladite matrice sans formation de gel.

6. Procédé selon la revendication 5, comprenant l'exposition de la surface d'adsorption de ladite matrice au fibrinogène en quantité suffisante pour couvrir efficacement ladite surface adsorbante et l'introduction subséquente de la thrombine pour convertir ledit fibrinogène adsorbé en fibrine, ce par quoi ladite surface adsorbante est efficacement complètement remplie par la fibrine adsorbée.

7. Procédé d'isolement d'activateur plasminogène selon la revendication 1, caractérisé par les étapes de:

— fourniture d'une matrice adsorbante solide avec de la fabrine par traitement de fibrinogène avec de la thrombine en présence de ladite matrice pour faire adsorber la fibrine sur le substrat de ladite matrice;
— l'exposition au substrat d'un liquide mère contenant de l'urokinase pour que les molécules qui ont une haute affinité vis-à-vis de la thrombine soient liées à la fibrine;
— l'élimination du matériau non lié par lavage avec une solution tampon;
— la séparation de l'urokinase à partir de la fibrine en éluant d'abord l'urokinase à partir de la surface de fibrine par un agent éluant comprenant un membre du groupe constitué d'arginine, de lysine et d'acide epsilon-amino caproïque et la séparation subséquence de ladite urokinase à partir dudit agent.

8. Procédé selon l'une quelconque des revendications 2 à 7, caractérisé en ce que la susdite matrice comprend des particules et en ce que le liquide est exposé à ladite matrice portant la fibrine par mélange desdites particules dans ledit liquide.

9. Procédé selon l'une quelconque des revendications 2 à 8, caractérisé en ce que le susdit liquide est éliminé par décantation et filtration suivis de lavages répétés avec le tampon.

10. Concentré d'enzyme d'activateur plasminogène produit par le procédé selon l'une quelconque des revendications 2 à 9.

11. Agent thérapeutique comprenant le concentré d'enzymes d'activateur plasminogène selon l'une quelconque des revendications 1 à 10.

12. Activateur plasminogène selon l'une quelconque des revendications 1, 10 ou 11 portant un marqueur qui permet à l'enzyme d'être utilisé comme sonde, par exemple pour détecter les caillots de sang.

13. Activateur plasminogène selon la revendication 12, caractérisé en ce que ledit marqueur est fourni par de l'iode radio-active ou du technetium lié au susdit enzyme.